# EUROPEAN PATENT APPLICATION

(11) **EP 1 277 459 A1**
(43) Date of publication of application: **22.01.2003**
(21) Application number: 01925901.9
(22) Date of filing: 25.04.2001
(51) Int. Cl.: A61K 7/09

(54) **PERMING COMPOSITIONS AND PERMING METHOD BY USING THE SAME**

(30) Priority: 28.04.2000 JP 2000128824
(71) Applicant: Ceramide Co., Ltd., Osaka-shi, Osaka 550-0002 (JP)
(72) Inventor: OGOH, Eiji, Ceramide Co., Ltd., Osaka-shi, Osaka 550-0002 (JP)
(74) Representative: Weber, Thomas, Dr.Dipl.-Chem.
(86) International application number: JP0103565
(87) International publication number: WO01082881

(57) **Abstract**

A permanent composition is prepared by aging an aqueous composition comprising a ceramide and cysteine by warming at 30 to 40 °C. The ratio of the ceramide to the cysteine may be 1/300 to 1/50 as a ceramide/cysteine ratio (weight ratio). The amount of the ceramide may be 0.01 to 1 % by weight, and the amount of cysteine may be 1.5 to 7.5 % by weight, relative to the total weight of the permanent composition. The permanent composition may further comprise a phospholipid. The permanent processing is carried out by washing a hair with a shampoo containing the ceramide, applying a treatment containing the ceramide to the hair, applying a mixed composition of the permanent composition and a treatment to the hair, winding the treated hair, and applying the permanent composition to the wound hair.

## Description

### TECHNICAL FIELD

The present invention relates to a permanent composition comprising a ceramide and cysteine, a process for producing the same, a set or kit of permanent-treating agents, and a permanent process, which are useful for permanent-shaping a hair.

### BACKGROUND ART

A hair comprises a keratin mainly composed of 18 species of amino acids, and the amino acids constituting the keratin contains about 16 % of cysteine. Cysteine forms cystine by disulfide-bonding to shape a fixed configuration of hairs. The permanent processing (especially, waved permanent processing) for the hair is carried out by treating the hair with a permanent composition (or a treating composition) containing a reducing agent for breakage or cleavage of a disulfide bond of keratin, forming the hair with the use of a rod, curler and the like having a desired configuration or shape with applying tension, and applying, to the curled hair, an oxidizing agent (an aqueous solution containing an agent such as a hydrogen peroxide, an alkali bromate, a perchlorate and a polythionate) to reconstruct disulfide bonds between cysteines and fix the disulfide bonds. Such a method is capable of processing a hair into a straightened-form or a curled-form.

The reducing agent of the permanent composition includes, for example, thioglycolic acid, cysteine, cysteamine, glycerylthioglycolate, thiolactic acid and the like are used. Typically, a permanent composition containing thioglycolic acid and a permanent composition containing cysteine are employed.

The permanent composition containing thioglycolic acid has an excellent permanent processability for hairs, but markedly damages the hair because of stronger activity of thioglycolic acid than that of cysteine. Thus, the application of the permanent composition is particularly restricted in case of processing a severe damaged hair such as a bleached hair and a dyed hair. Moreover, when thioglycolic acid is used, the permanent shaping degree of the hair is different between the hair roots and the hair roots, and the composition causes frequently a dried and loosed hair, a split hair, a notched hair or the like.

Although the permanent composition containing cysteine hardly damages hairs, the permanent composition is inferior in the permanent processability. Therefore, it is difficult to wind hairs on conventional rods, and small rods are mainly required to curl or wave hair, and thus the use of the permanent composition containing cysteine is restricted to specific applications. Moreover, a permanent processing for a severely damaged hair is much more difficult than that for a healthy hair. Moreover, the processing with the use of the permanent composition containing cysteine increases a split hair-roots and a notched hair, and disappears luster of hair. Further, even when cysteine which seems to hardly damage hair is used, after several times of permanent processing with the cysteine, the permanent shaping is unable to realize, because it seems probably that the cysteine is flaked inside the hair by drying the hair. The Japanese Patent Application Laid-Open No. 500147/1999 (JP-11-500147A) discloses an oxidation composition comprising a ceramide-type compound and an oxidizing agent such as an aqueous hydrogen peroxide, and a hair-bleaching or permanent processing method with the use of the oxidation composition. However, this method also deteriorates permanent formability or shapability in case of a severely damaged hair or a hair damaged by permanent processing.

It is, therefore, an object of the present invention to provide a permanent composition, a process for producing the same, a set (combination) of permanent-treating agents, and a permanent process, which have an excellent permanent shapability or processability without damaging hair.

It is another object of the present invention to provide a permanent composition, a process for producing the same, a set of permanent-treating agents, and a permanent process, which are capable of making permanent shaping or waving even for a severe damaged hair which seems to have difficulty of a permanent shaping and a hard-cuticle hair.

It is further another object of the present invention to provide a permanent composition, a process for producing the same, a kit of permanent-treating agents, and a permanent process, which inhibit or suppress a damage of a hair, and rather improve the permanent shapability or formability, even if a permanent processing is carried out repeatedly.

It is still another object of the present invention to provide a permanent composition, a process for producing the same, a kit of permanent-treating agents, and a permanent process, which are capable of safely forming a permanent shaping or configuration even for the subject having a sensitive skin etc.

### DISCLOSURE OF INVENTION

The inventors of the present invention made intensive studies to achieve the above objects and finally found that the use of a permanent composition comprising a combination of a ceramide and cysteine gives effectively an excellent permanent shapability or formability without damaging hair. The present invention was accomplished based on the above findings.

That is, the permanent composition of the present invention comprises a ceramide and cysteine. The ratio of the ceramides to cysteine may be 1/1000 to 5/1 as a ceramides/cysteine ratio (weight ratio), and the permanent composition may comprise 0.001 to 5 % by weight of the ceramides and 1 to 10 % by weight of cysteine. The permanent composition may further comprise a phospholipid.

Moreover, the present invention includes a set or kit of permanent-treating agents comprising the permanent composition recited above and at least one treating agent selected from the group consisting of a shampoo, a treatment, an oxidizing agent, a rinse (rinsing agent) and a cuticle-conditioning (cuticle-repairing) agent, wherein the treating agent comprises ceramides. The treating agent may further comprise a phospholipid.

Moreover, the present invention also discloses a process for producing the permanent composition, which comprises aging an aqueous composition comprising ceramides and cysteine. The aging of the aqueous composition may be carried out by warming the composition at about 25 to 50 °C.

Furthermore, the present invention includes a permanent process which comprises applying the permanent composition to a hair. The permanent process of the present invention may comprise washing a hair with a shampoo, applying the permanent composition to the washed hair, and forming the treated hair into a desired shape or configuration. Moreover, the permanent process of the present invention may comprise applying the permanent composition and a treatment containing a ceramide to a hair, forming the treated hair into a desired shape or configuration, if necessary, further applying the permanent composition to the formed hair. The permanent process of the present invention may comprise washing a hair with a shampoo containing a ceramide, applying a treatment containing a ceramide to the washed hair, applying the permanent composition to the treated hair, forming the treated hair into a desired shape or configuration, and treating the shaped hair with an oxidizing agent.

The term "ceramide" means not only a ceramide but also a glycoceramide, a pseudoceramide and a lipid comprising these compounds or substances.

### BEST MODE FOR CARRYING OUT THE INVENTION

### [A permanent composition]

The feature of a permanent composition of the present invention resides in that a combination of a ceramide with cysteine improves permanent formability regardless of species and quality or grade of hair without damaging hair.

As the ceramides, there may be mentioned a ceramide derived or originated from natural products, for example, a ceramide derived or originated from plants (e.g., wheat, soybean, rice), a ceramide derived or originated from animals (e.g., keratin intercellular lipid of human's skin), a ceramide derived or originated from microorganisms (e.g., yeast), and a synthetic ceramide obtainable by chemical synthesis. The ceramides further include a ceramide in which a fatty acid (including hydroxy acid) is amide-bonded to a sphingosine. The ceramides may be ceramide 1, ceramide 2, ceramide 3, ceramide 4, ceramide 5, ceramide 6I, ceramide 6II or the like. For example, the ceramide is represented by the following formula (I): 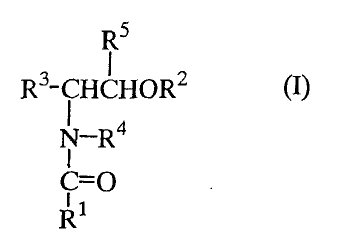 wherein R¹ represents a C₁₋₅₀hydrocarbon group which may have a substituent, group -R⁶-(NR⁷-CO)-R⁸
wherein R⁶ and R⁸ each represents a C₁₋₃₀ hydrocarbon group which may have a substituent,
and R⁷ represents hydrogen atom or a C₁₋₂₀ hydrocarbon group which may have a substituent,
or group -R⁹-O-CO-R¹⁰
wherein R⁹ represents a C₁₋₂₀hydrocarbon group,
R¹⁰ represents a C₁₋₁₂hydrocarbon group;
R² represents hydrogen atom, (poly)glycosyl group, sulfuric acid (or a salt thereof) group, sulfonic acid (or a salt thereof) group, phosphoric acid (or a salt thereof) group, or phosphoryl (or a salt thereof) group; R³ represents hydrogen atom, or a C₁₋₃₃hydrocarbon group which may have a substituent; R⁴ represents hydrogen atom, a C₁₋₅₀hydrocarbon group which may have a substituent, or -CH₂-CH(OH)-CH₂-O-R¹¹ group wherein R¹¹ represents a C₁₀₋₂₆ hydrocarbon group; and R⁵ represents hydrogen atom, or a C₁₋₃₀hydrocarbon group which may be a substituent.

In the formula (I), the preferred substituent R¹ includes a C₁₋₅₀hydrocarbon group or group -R⁹-O-CO-R¹⁰. As the C₁₋₅₀hydro carbon group (preferably C₅₋₅₀hydrocarbon group) represented by R¹, there may be mentioned a linear or branched and saturated or unsaturated hydrocarbon group, for example, a residue of a linear or branched saturated fatty acid such as butyric acid, caproic acid, caprylic acid, capric acid, lauric acid, myristic acid, palmitic acid, stearic acid, arachidic acid, and behenoic acid; a residue of a linear or branched unsaturated fatty acid such as myristoleic acid, palmitoleic acid, petroselinic acid, oleic acid, linolic acid, and linolenic acid and the like. The preferred R¹ is an aliphatic C₁₀₋₂₄hydrocarbon group. R¹ may have a substituent, for example, a hydroxyl group and the like, and the hydroxyl group may be further esterified with a C₁₋₃₅fatty acid (especially, higher C₁₀₋₂₄fatty acid) which may have a substituent.

The preferred substituent R⁶ includes a linear or branched and saturated or unsaturated aliphatic hydrocarbon group, for example, a C₅₋₂₀alkylene group. Preferable examples of the substituent R⁷ are hydrogen atom, or a linear or branched and saturated or unsaturated aliphatic hydrocarbon group, for example, a C₁₋₁₀alkyl group, and particularly a C₁₋₆alkyl group. As the substituent R⁸, there are preferably exemplified a linear or branched and saturated or unsaturated aliphatic hydrocarbon group, for example, a C₅₋₂₀alkyl group. The total carbon number of R⁶ and R⁸ is about 9 to 30.

The substituent R⁹ includes preferably a linear or branched and saturated or unsaturated aliphatic hydrocarbon group, for example, a C₁₀₋₂₀alkylene group. The substituent R¹⁰ is preferably a linear or branched and saturated or unsaturated aliphatic hydrocarbon group, for example, a C₁₋₁₂alkyl group.

The substituent R² includes preferably hydrogen atom or (poly)glycosyl group. As the (poly)glycosyl group of R², there may be mentioned, for example, glycosyl group or a dimer to tetramer thereof, galactosyl group or a dimer to octomer, sulfogalactosyl group, mannosyl group, fructosyl group, sorbosyl group and the like. The phosphoryl (or a salt thereof) group includes phosphoryl-alkylamine groups such as phosphorylethylamine group, phosphorylalkylammonium groups such as phosphorylethylammonium group and the like.

The substituent R³ includes preferably a linear or branched and saturated or unsaturated aliphatic hydrocarbon group, for example, a fatty acid residue similar to R¹ (especially, an aliphatic C₁₀₋₂₄hydrocarbon group). The substituent R³ may have, for example, a hydroxyl group (especially, α-hydroxyl group), a C₁-₁₄alkyl group or the like, and the hydroxyl group may be esterified with an inorganic acid or a C₁₋₃₅fatty acid which may have a substituent (e.g., α-hydroxyl group)(in particular, a higher C₁₀₋₂₄fatty acid which may have α-hydroxyl group), or may be etherified with (poly)glycosyl group, sulfuric acid (or a salt thereof) group, sulfonic acid (or a salt thereof) group, phosphoric acid (or a salt thereof) group, or phosphoryl (or a salt thereof) group.

The substituent R⁴ includes preferably hydrogen atom, or a linear or branched and saturated or unsaturated aliphatic C₁₋₃₀hydrocarbon group, for example, a C₁₋₂₄alkyl group (in particular, C₁₋₁₂alkyl group). The substituent R⁴ may have a hydroxyl group or the like and the hydroxyl group may be esterified with a C₁₋₃₅fatty acid (preferably a higher C₁₀₋₂₄fatty acid).

The substituent R¹¹ includes a linear or branched and saturated or unsaturated aliphatic hydrocarbon group, for example, a C₁₀₋₂₆alkyl group.

The substituent R⁵ preferably includes hydrogen atom, or a linear or branched and saturated or unsaturated aliphatic hydrocarbon group, for example, a C₁₋₁₀alkyl group, and particularly a C₁₋₆alkyl group. The substituent R⁵ may have a hydroxyl group, and the hydroxyl group may be further etherified with (poly)glycosyl group, sulfuric acid (or a salt thereof) group, sulfonic acid (or a salt thereof) group, phosphoric acid (or a salt thereof) group, or phosphoryl (or a salt thereof) group.

Among these ceramides, a ceramide represented by the following formula (II) is usually employed: 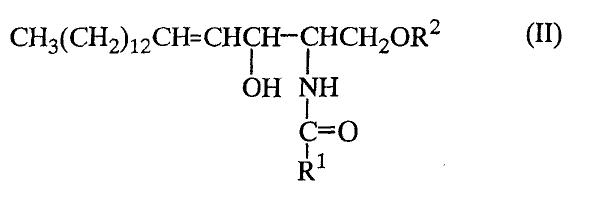 wherein R¹ represents a linear or branched and saturated or unsaturated C₁₋₃₀hydrocarbon group (preferably a C₁₀₋₂₄alkyl group) and R² represents hydrogen atom or a glycosyl group.

As the ceramides, there may be mentioned, for example, 2-N-linoleoylaminooctadecane-1,3-diol, 2-N-oleoylaminooctadecane-1,3-diol, 2-N-palmitoylamino octadecane-1,3-diol, 2-N-stearoylaminooctadecane-1,3-diol, 2-N-behenoylaminooctadecane-1,3-diol, 2-N-(2-hydroxypalmitoyl)aminooctadecane-1,3-diol, 2-N-stearoylaminooctadecane-1,3,4-triol, N-stearoylphyto sphingosine, 2-N-palmitoylaminohexadecane-1,3-diol, bis(N-hydroxyethyl-N-cetyl)malonamide, N-(2-hydroxy ethyl)-N-(3-cetyloxy-2-hydroxypropyl)cetylamide and so on.

As the ceramides, a ceramide extructed from naturally occurring or originated plants (e.g., wheat-germ oil) is preferred. This ceramide is represented by the formula (II) wherein R¹ is a C₁₀₋₃₀alkyl group, a hydroxy-C₁₀₋₃₀alkyl group, or a saturated or unsaturated C₁₀₋₂₄ aliphatic acyloxyC₁₀₋₃₀alkyl group; and R₂ is glucosyl group.

These ceramides can be used singly or in combination.

The proportion of the ceramides in the permanent composition can be selected within the range of about 0.001 to 5 % by weight, and is usually about 0.005 to 3 % by weight (e.g., about 0.01 to 3 % by weight), and preferably about 0.01 to 1 % by weight. Incidentally, when the ceramides are included in the wheat-germ oil, the proportion of the wheat-germ oil is, depending on the content of the caramides in the wheat-germ oil, usually about 0.01 to 5 % by weight, preferably about 0.05 to 5 % by weight, and particularly about 0.05 to 3 % by weight in the amount of the permanent composition. The present invention can effectively realize a curled- or waved-hair from a tip to a root, compared with a conventional cysteine-containing permanent composition.

As the cysteine, conventional one as a reducing agent for a permanent composition can be used. The ratio of the cysteine in the permanent composition is usually not more than 10 % by weight (e.g., about 1 to 10 % by weight), and preferably about 1 to 7.5 % by weight (e.g., about 1.5 to 7.5 % by weight). Incidentally, a permanent composition containing cysteine in an amount of about 1.5 to 5.5 % by weight is capable of making or forming a cold permanent with warming at a low temperature, and a permanent composition containing cysteine in an amount of about 3.5 to 7.5 % by weight realize a cold permanent without warming.

The ratio (weight ration) of the ceramide relative to cysteine can be selected, depending on the degree of damage of hair, the grade or quality of hair owing to the age etc and the like, and can be selected within the range, for example, the ceramide/cysteine = about 1/1000 to 5/1 (e.g., about 1/750 to 1/1), preferably about 1/500 to 1/1 (e.g., about 1/500 to 1/30), and more preferably about 1/300 to 1/50. For example, for a lowly damaged-hair, the ratio (weight ration) of the ceramide to cysteine can be selected within the range of the ceramide/cysteine = about 1/300 to 1/100 (e.g., about 1/250 to 1/100), and for a heavily damaged-hair, the ratio (weight ration) of the ceramide to cysteine can be selected within the range of the ceramide/cysteine = about 1/150 to 1/50 (e.g., about 1/100 to 1/50).

In the present invention, the composition comprising the ceramides and cysteine may be used in combination with a phospholipid. The combination of the phospholipid and the ceramides promotes efficiently a penetration of cysteine into cells, and so permanent can be carried out effectively.

As the phospholipid, there may be mentioned, for example, a phospholipid derived or originated from naturally occurring products, for example, plants (e.g., seeds of soybeans, peas), animals (e.g., egg yolk, butterfat, organs such as liver, muscle, marrow, brain), microorganisms (e.g., yeast, bacterium) or the like, and may be mentioned a synthetic phospholipid obtained by chemical syntheses. The phospholipid includes a glycerophospholipid and a sphingophospholipid. As the glycerophospholipid, there are exemplified phosphatidylcholine (lecithin), phosphatidylethanolamine (kephalin), phosphatidylserine (cephalin), phosphatidylinositol (inositolphospholipid), alkoxyphospholipid (α-glycerylether), phosphatidylglycerol, phosphatidalethanolamine (plasmalogen) or the like. The sphingophospholipid includes, for example, sphingomyelin, sphingoethanolamine or the like. Among these phospholipids, the glycerophospholipid is preferred. For example, the preferred glycerophospholipid is represented by the following formula (III); 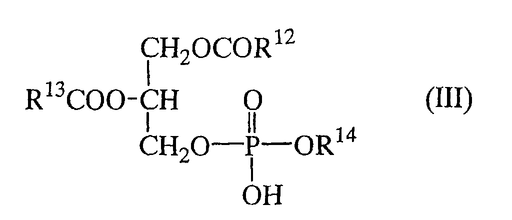 wherein R¹² and R¹³ each represents a C₁₋₅₀ hydrocarbon group which may have a substituent, and R¹⁴ represents an aminoalcohol residue or a sugar or saccharide residue.

In the formula (III), example of the hydrocarbon group of the R¹² and R¹³ includes the hydrocarbons similar to the R¹. As the substituents R¹² and R¹³, it is preferred that a residue of a saturated or unsaturated linear or branched fatty acid such as capric acid, lauric acid, myristic acid, palmitic acid, stearic acid, arachidic acid, behenic acid, linoleic acid, linolenic acid, eleostearic acid, and arachidonic acid. In the substituents R¹² and R¹³, the ratio (mole ratio) of the saturated fatty acid residue to the unsaturated fatty acid residue is the saturated fatty acid residue/the unsaturated fatty acid residue = about 100/0 to 50/50, preferably about 99/1 to 70/30, and more preferably about 97/3 to 80/20 (particularly about 95/5 to 85/15). The substituents R¹² and R¹³ usually includes a residue of C₁₄₋₂₀ linear saturated fatty acid such as stearic acid and palmitic acid of not less than 80 mole % (particularly about 85 to 95 mol %). Example of the substituent R¹⁴ includes an aminoalcohol residue (e.g., choline residue, ethanolamine residue, serine residue), a sugar or saccharide residue (e.g., myoinositol residue, galactose residue) or the like.

Such a glycerophospholipid (III) includes, for example, lecithin, kephalin, cephalin, and inositolphospholipid. These glycerophospholipids (III) can be used singly or in combination. Among these glycerophospholipids (III), particularly the lecithin represented by the formula (IV) including further particularly lecithins derived from soybean oil or egg yolk, is generally used; 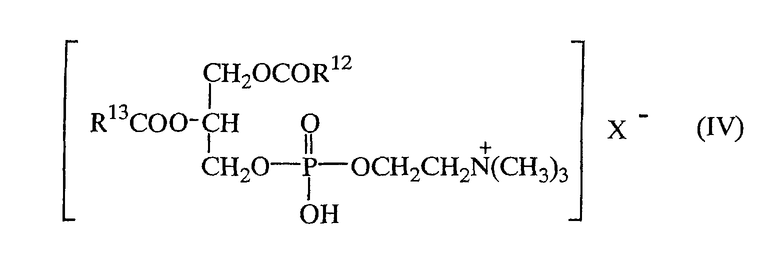 wherein X⁻ represents anion and R¹² and R¹³ have the same meanings as defined above.

In the formula (IV), as the anion of X , a hydroxy ion (OH⁻), a halogen ion (iodine, bromine, chlorine, fluorine ions), a mono- or poly-basic carboxylic acid ion (e.g., acetic acid ion, maleic acid ion), a hydroxycarboxylic acid ion (e.g., tartaric acid hydrogen ion, citric acid hydrogen ion, gluconic acid ion) or the like.

When the phospholipid have the unsaturated fatty acid residue, the phospholipid may be hydrogenated to improve the stability, and an unsaturated units of hydrocarbon group of the phospholipid may be hydroxylated to improve the water solubility.

The phospholipid can be used singly or in combination.

The ratio of the phosphalipid can be selected within the range of about 0.001 to 15 % by weight, and is usually about 0.005 to 10 % by weight (e.g., about 0.01 to 5 % by weight), and preferably about 0.01 to 3 % by weight (particularly about 0.1 to 1 % by weight) in the permanent composition.

The ratio of the ceramides to the phospholipid may be the ceramides/the phospholipid = about 1/0.002 to 1/15000, preferably about 1/0.005 to 1/1000, and more preferably about 1/0.01 to 1/500 (particularly about 1/0.1 to 1/50).

The permanent composition is usually water soluble, and contains water as a predominant solvent. The pH value of the permanent composition is not particularly restricted to a specific value or range so far as the hair would not be damaged or injured, and is, for example, about 8 to 11, and preferably about 9 to 10.

To the permanent composition can be added other additives such as an alcohol (e.g., C₁₋₁₀alcohols such as ethanol, isopropanol, and butanol), an amino acid or a salt thereof (e.g., cysteine, arginine), a preservative or antiseptic (e.g., p-hydroxybenzoate), a sequestering agent (e.g., edetates such as disodium edetate and tetrasodium edetate), a buffer, a quaternary ammonium salt or preserving agent, a cationic polymer, an opacifier, a reducing agent (e.g., thioglycolic acid or salts thereof), a moisturizing agent (e.g., ethylene glycol, polyethylene glycol, propylene glycol, polypropylene glycol, ethylene oxide-propylene oxide copolymers, polyhydric alcohols such as glycerin, C₁₂₋₂₀alcohols such as lauryl alcohol, cetanol, and stearyl alcohol), a surfactant (e.g., nonionic surfactants, anionic surfactants, cationic surfactants, amphoteric surfactants), a pH controlling agent (pH adjuster) (e.g., ammonia, alkanolamines such as monoethanolamine), an acid (e.g., organic carboxylic acids such as acetic acid, organic polybasic-carboxylic acids such as maleic acid, hydroxycarboxylic acids such as lactic acid and citric acid), a hydrolyzed protein (e.g., hydrolyzed silk liquid, hydrolyzed collagen powder), water-soluble polymers (e.g., cellulose derivatives such as hydroxyethyl cellulose and carboxymethyl cellulose, polyvinyl alcohol, polyvinyl pyrrolidone).

### [Production Process of the permanent composition]

The permanent composition of the present invention can be obtained by aging an aqueous composition comprising a ceramide and cysteine. An aging or ripening of the aqueous composition may be carried out by storage of the composition at a lower temperature (e.g., about 10 to 20 °C) for a long period of time (e.g., not less than one week), and the aging is preferably carried out by warming the composition at about 25 to 50 °C, preferably about 25 to 40 °C, and more preferably about 30 to 40 °C to develop or express a high potency in a shorter time. The aging with warming may be carried out or conducted, for example, for about 10 minutes to 7 days, and preferably 30 minutes to 3 days (e.g., about 1 to 12 hours). The aging may be conducted under an opened or closed system in an oxygen-containing gas atmosphere (e.g., air atmosphere) or an inert gas with allowing to stand or stirring. Incidentally, in a conventional permanent composition, the warming or heating is incompatible with permanent shapability since the warming results in a permanent difficulty. Meanwhile, according to the permanent composition of the present invention, the aging with warming or heating at the temperature mentioned above expresses or shows a high permanent shapability to form a sufficient curl and wave. Regarding the aging, an aqueous composition comprising at least ceramides and cyateine may be aged, and may be aged together with additives (e.g., phospholipid).

### [Permanent Process]

The permanent process of the present invention usually comprises a hair-washing (shampooing) step, a hair-treatment step, a reducing and forming (or shaping) step, an oxidizing step, and a rinsing step. Incidentally, the hair-treatment step is not essentially needed in the permanent for a virgin hair or the similar hair.

In the hair-washing (shampooing) step, a shampoo excluding ceramides may be used as a shampoo, and a shampoo containing or comprising ceramides is preferably used. Moreover, for a hair which has been severely damaged, a cuticle-conditioning agent comprising a keratin (particularly both of a keratin and ceramides) may be applied to hairs for coating, or a tip of severely damaged-hair may be cut after washing the hair.

The proportion of the ceramides in the shampoo may be about 0.001 to 5 % by weight, and preferably about 0.005 to 3 % by weight. The shampoo may comprise, besides surfactants, a conventional component (e.g., perfumes, colorants, moisturizing agents, sequestering agents, preservatives, amino acids or salts thereof, hydrolyzed proteins). Particularly, the shampoo contains preferably the phospholipid in an amount of about 0.005 to 10 % by weight, preferably about 0.01 to 5% by weight, and more preferably about 0.01 to 3 % by weight (particularly about 0.1 to 1% by weight).

The proportion of ceramides in the cuticle-conditioning agent may be about 0.001 to 5 % by weight, and preferably about 0.005 to 3 % by weight. The cuticle-conditioning agent may comprise, besides the keratin, a conventional component (e.g., alcohols, preservatives, buffers, surfactants, quaternary ammonium salts or preserving agents, cationic polymers, amino acids or salts thereof, hydrolyzed proteins). Particularly, it is preferred that the cuticle-conditioning agent contains the phospholipid in an amount of about 0.005 to 10 % by weight, preferably about 0.01 to 5% by weight, and more preferably about 0.01 to 3 % by weight (particularly about 0.1 to 1% by weight).

In the hair-treatment step, after washing the hair, a treatment containing ceramides may be further applied to the hair for coating. Particularly, the step is effective for a hair which has been severely damaged. The ratio of the ceramides in the treatment can be selected within the range of about 0.001 to 5 % by weight, and preferably about 0.005 to 3 % by weight. The treatment may comprise a conventional component (e.g., perfumes, colorants, thickening agents, preservatives, quaternary ammonium salts or preserving agents, cationic polymers, buffers, moisturizing agents, surfactants, amino acids or salts thereof, hydrolyzed proteins). Particularly, it is preferred that the amount of the phospholipid in the treatment is about 0.005 to 10 % by weight, preferably about 0.01 to 5% by weight, and more preferably about 0.01 to 3 % by weight (particularly about 0.1 to 1% by weight).

Incidentally, in a conventional permanent process, an application of the treatment to the hair for coating before a permanent processing has not been conducted positively because of inhibiting the formation of a curl or wave. According to the process of the present invention, the application of the treatment containing ceramides to hairs for coating promotes the formation of curl or wave, and therefore it is preferred to use the treatment containing ceramides.

After the hair-treatment step, if necessary, a cuticle-conditioning or cuticle-repairing agent may be applied to the hair for coating, and/or water may be sprayed on the hair.

In the reducing and forming or fashioning step, the treated hair is formed in a desired configuration with acting a tension on the hair, and is treated with a reducing agent. The forming step can be conducted by, for example, rolling or winding the hair on a desired-form rod, curler or the like under tension (a winding procedure) or by straightening the hair. Usually, before the forming step including winding, disulfide bonds between cysteines of keratins in the hair are broken or cleaved by applying the reducing agent (e.g., the permanent composition containing cysteine) to the hair for coating.

The coating or application of the permanent composition may be carried out before and/or after the forming step for forming a desirable hair-configuration. In particular, when an application including coating is conducted before the forming step, it is preferred that a composition containing a lower concentration of cysteine is applied to the hair for coating, and such composition includes, for example, a composition containing ceramides and cysteine in a ratio of the ceramides/cysteine = about 1/250 to 1/100 (weight ratio) (e.g., a mixture of the hair-permanent composition and the treatment containing ceramides). When the application including coating is carried out after the forming step, it is preferred that a composition containing a higher concentration of cysteine is applied to the hair for coating, and such composition includes, for example, a composition comprising ceramides and cysteine in a ratio of the ceramides/cysteine = about 1/100 to 1/50 (weight ratio).

After forming the hair, the hair may be irradiated by a far infrared ray. When the hair has been severely damaged, the hair may be irradiated by a far infrared ray with a low temperature, or may be warmed. Moreover, after the irradiation of the far infrared ray, the hair may be warmed.

Subsequently, in the oxidizing step, an oxidizing agent is applied to the hair for coating in order to reconstruct the disulfide bonds between cysteines. As the oxidizing agent, a conventional oxidizing agent can be used, and there may be mentioned, for example, an aqueous solution of a hydrogen peroxide, an alkali bromate, a perchlorate, and a polythionate. The pH of the treating composition comprising the oxidizing agent may be about 5 to 8, and preferably about 6 to 7.5. The oxidizing agent may be applied to the hair plural times for coating. The oxidizing agent may further comprise, besides oxidants, a conventional component (e.g., sequestering agents, surfactants, moisturizing agents, preservatives, pH controlling agents).

After the forming step, in the rinsing step, usually the hair is coated with a rinse, and the coated hair was washed. Among rinses, a rinse containing ceramides is preferably used. The proportion of the ceramides in the rinse may be about 0.001 to 5 % by weight, and preferably about 0.005 to 3 % by weight. The pH value of the rinse is about 1 to 4, and preferably about 2 to 3. The rinse may further comprise a conventional component (e.g., alcohols, perfumes, colorants, preservatives, surfactants, amino acids or salts thereof, hydrolyzed proteins, pH adjusters). Particularly, it is preferred that the rinse comprises the phospholipid in an amount of about 0.005 to 10 % by weight, preferably about 0.01 to 5 % by weight, and more preferably about 0.01 to 3 % by weight (particularly about 0.01 to 1 % by weight).

In the process of the present invention, it is essentially required to use at least the permanent composition for a permanent treatment or processing, and, if necessary, at least one treating agent selected from the group consisting of a shampoo, a treatment, a cuticle-conditioning agent, a rinse and the like may be combined with ceramides for the permanent treatment. Particularly, a combination of the permanent composition and the treating agent comprising ceramides (in particular, both of the ceramides and a phospholipid) improves effectively the permanent shapability even if hair has been damaged or injured.

The damaged hair can be frequently found in a bleached hair and/or a dyed hair. Among the damaged hairs, a particularly severely damaged hair is called as a porous hair in which a filling material in hairs is removed off to form a hole. The ceramides fill up the holes of the damaged hair to normalize the damaged hair, and therefore a permanent shapability or formability can be enhanced. It is particularly effective to use a shampoo, a treatment, or a cuticle-adjusting agent, which comprise the ceramides, for a damaged human hair of middle-aged or more-aged and a damaged hair of a limp hair or a catty hair. Therefore, the present invention also discloses a set or kit of permanent-treating agents comprising a permanent composition and at least one treating agent selected from the group consisting of a shampoo, a treatment, an oxidizing agent, a rinse and a cuticle-conditioning agent, wherein the treating agent comprises a ceramide (in particular both of the ceramide and a phospholipid).

Further, although a conventional permanent composition damages or injures a hair whenever the permanent treatment is carried out, the present invention inhibits or suppresses effectively the damage or injury of the hair, even if the permanent treatment is repeated, and becomes the hair thick and healthy by the ceramides to improve the permanent shapability or formability of the hair whenever the permanent treatment is carried out repeatedly. That is, although a cysteine-series permanent composition gives a low damage to the hair compared with thioglycolic acid, the permanent realization cannot be achieved after several permanent treatments with the use of the cysteine-series permanent composition. Contrarily, the process of the present invention effectively prevents a flaking of cysteine to realize a high permanent shapability repeatedly because of comprising the ceramides.

Moreover, compared with Japanese hairs, westerner's hairs has a lower water content in the cuticle, a higher stiffness or rigidity, and much oil content, and therefore it is difficult to form a desired curl and wave configuration for the westerner's hairs by a conventional methods. On the other hand, the method of the present invention sufficiently forms the curl and wave for the westerner's hairs and the similar hair regardless the quality or grade of the cuticle.

In the present permanent method, the steps mentioned above can be suitably combined dependent on the degree of damage of hair. For example, for a hair which has been severely damaged, a shampoo and/or a treatment each comprising the ceramide may be used, and a permanent composition having a lower cysteine content, a cuticle-conditioning agent containing the ceramide, or a rinse containing the ceramide is preferably used. Moreover, drying at a low temperature without irradiation of a far infrared ray, hair-cutting of a tip of severely damaged hairs and the other processes may be preferably carried out.

### INDUSTRIAL APPLICABILITY

According to the present invention, an excellent permanent treatment can be realized without damaging hair. Moreover, the permanent treatment can be conducted for a severely damaged-hair having difficulty of the permanent shaping. Moreover, the hair is prevented effectively from damage even though the permanent treatment is repeatedly given, and rather the permanent shapability or formability of the hair can be improved. Further, the permanent treatment can be safely conducted to a human person having a sensitive or tender skin (e.g., skin liable to be suffered from atopic dermatitis).

### EXAMPLES

The following examples are intended to describe this invention in further detail and should by no means be interpreted as defining the scope of the invention.

Incidentally, components of treating compositions used in the following examples are shown in Table 1 to 7. In the wheat-germ oil comprised in the treating compositions, the ceramide represented by the formula (II) is comprised therein in an amount of about 6 % by weight.

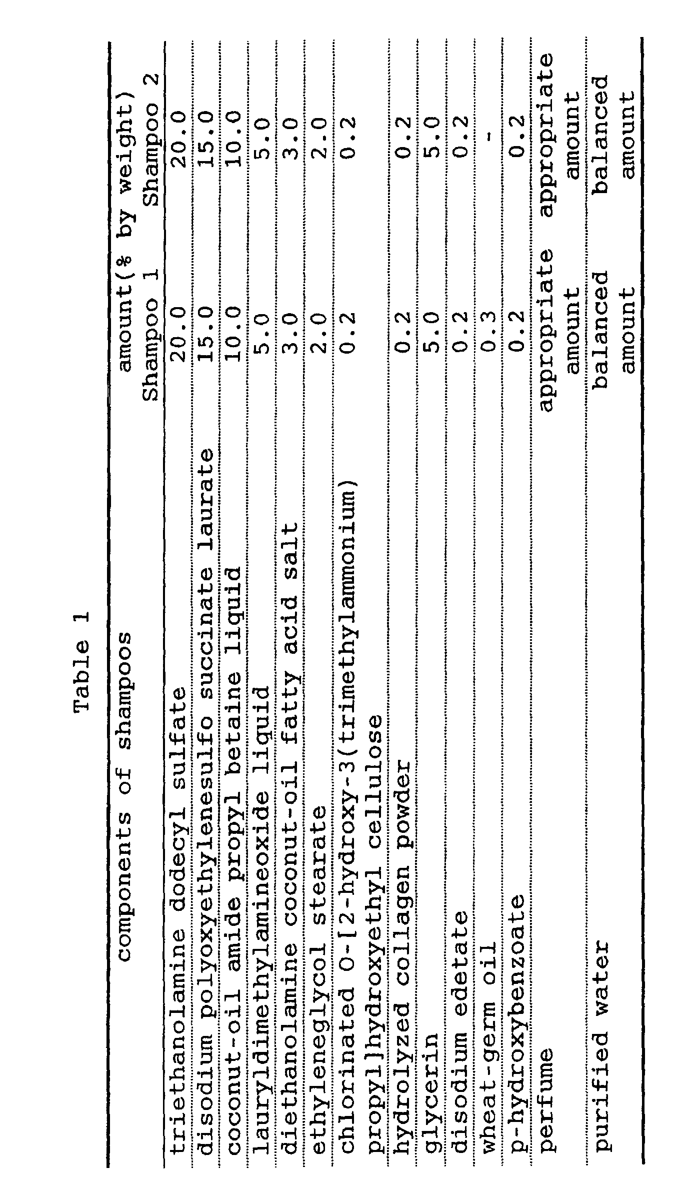

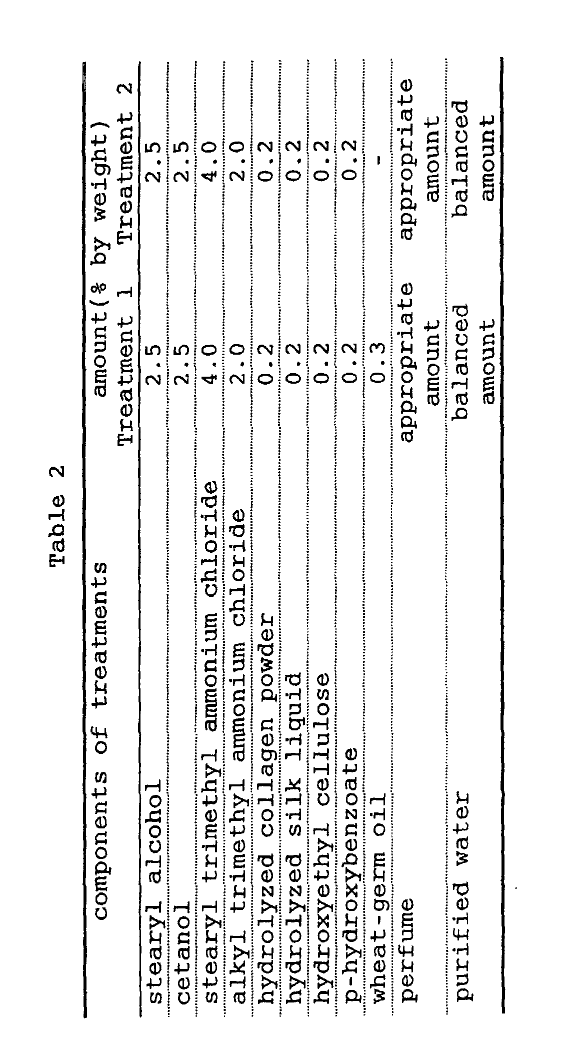

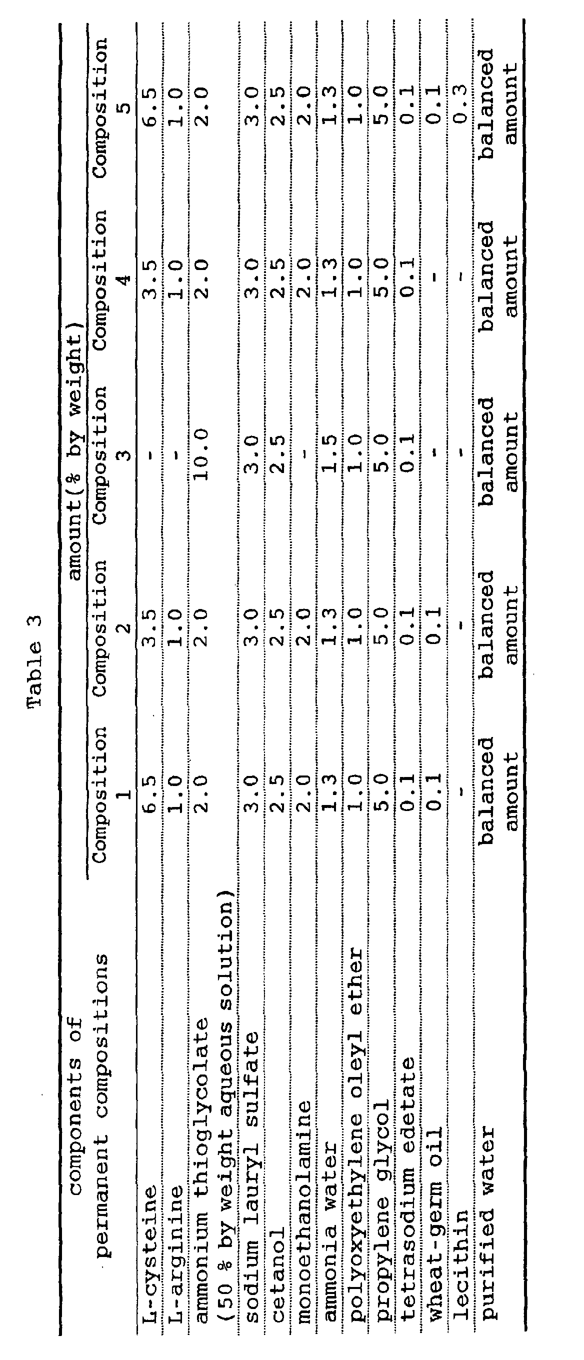

**Table 4**

| components of oxidizing agents | amount (% by weight) | |
|---|---|---|
| | Oxidizing agent 1 | Oxidizing agent 2 |
| sodium bromate | 9.0 | 7.5 |
| tetrasodium edetate | 0.2 | 0.2 |
| cetanol | 2.5 | 2.5 |
| trimethylammonium cetyl chloride | 2.0 | 2.0 |
| propyleneglycol | 3.0 | 3.0 |
| p-hydroxybenzoate | 0.2 | 0.2 |
| purified water | balanced amount | balanced amount |

**Table 5**

| components of Rinse | amount (% by weight) |
|---|---|
| ethanol | 3.0 |
| polyoxyethylene polyoxypropylene decyl tetradecyl ether | 3.0 |
| hydrolyzed collagen liquid | 0.2 |
| N-coconut-oil futty acid acyl triethanolammonium L-glutamate | 5.0 |
| citric acid | 0.1 |
| lactic acid | 0.1 |
| wheat-germ oil | 0.3 |
| p-hydroxybenzoate | 0.2 |
| purified water | balanced amount |

**Table 6**

| components of Intermediate rinse | amount (% by weight) |
|---|---|
| ethanol | 3.0 |
| polyoxyethylene polyoxypropylene decyl tetradecyl ether | 3.0 |
| hydrolyzed collagen liquid | 0.2 |
| N-coconut-oil futty acid acyl triethanolammonium L-glutamate | 5.0 |
| citric acid | 0.1 |
| lactic acid | 0.1 |
| p-hydroxybenzoate | 0.2 |
| purified water | balanced amount |

**Table 7**

| components of Cuticle-conditioning agent | amount (% by weight) |
|---|---|
| ethanol | 10.0 |
| polyoxyethylene polyoxypropylene decyl tetradecyl ether | 3.0 |
| hydrolyzed keratin liquid | 0.2 |
| stearyl trimethyl ammonium chloride | 5.0 |
| hydrolyzed silk liquid | 0.2 |
| wheat-germ oil | 0.3 |
| p-hydroxybenzoate | 0.2 |
| purified water | balanced amount |

### Example 1

A virgin hair of 10's (teen-aged) female was washed with 3 to 5ml of Shampoo 1, and the washed hair was further washed by 3 to 5ml of Shampoo 1 with fully lathering up. Subsequently, 1ml of Treatment 1 was applied to the washed hair for coating, and the treated hair was blotted with a towel to dry without rinsing of the Treatment 1. The hair was then coated by 9ml of the Treatment 1 again, the treated hair was sprayed successively with 100ml of water sufficiently and 10 to 20 ml of Cuticle-conditioning agent. Subsequently, 40 ml of a mixed composition of Permanent composition 1 (prepared by warming at 35 °C) and Treatment 1 in a ratio of the former/the latter = 4/1 (volume ratio) was applied to the hair for coating, and the coated hair was wound on a permanent rod (rolled on a permanent rod). The wound hair was sprayed with 20ml of water, and 40ml of Permanent composition 1 prepared by warming at 35 °C was applied to the hair for coating. The hair was covered with a cap, and was irradiated by a far infrared ray with a low temperature for 8 to 25 minutes. The curl was confirmed with visual confirmation by releasing one of rods from the hair. Subsequently, after 40 ml of water was freely sprayed on the hair, the hair was blotted with a towel slightly, and 40ml of Oxidizing agent 1 was applied to the hair for coating and allowed to stand for 5 minutes, and 40ml of the Oxidizing agent 1 was further applied to the hair for coating and left for 5 to 7 minutes. The hair was coated with 60 to 100ml of Rinse, after allowing to stand for an appropriate time, the permanent rods was removed from the treated hair. As a result, the curled permanent hair was firmly formed from the roots to the tips , and the formation of the curl (form stability) was also retained or lasted. Only 15 to 18 minutes were required to irradiate the hair with a far infrared ray at a low temperature for forming permanent waves.

### Example 2

The permanent procedures were conducted in the same manner as that in Example 1 except for using Permanent composition 1 aged at room temperature for 1 week instead of Permanent composition 1 prepared by warming at 35 °C. The resulting curl was excellently formed in the same shorter irradiating time of a far infrared ray as that in Example 1.

### Example 3

The permanent procedures were carried out in the same manner as that in Example 1 except for using Permanent composition 1 prepared without warming instead of Permanent composition 1 prepared by warming at 35 °C. As a result, it was required to irradiate the hair with a far infrared ray at a low temperature for not less than 25 minutes to form a wave, and the wave was deformed or collapsed.

### Comparative Example 1

A virgin hair of 10's female was washed with Shampoo 2, and the washed hair was hair-cut. Subsequently, 40 ml of Permanent composition 3 was applied to the hair by a spray gun for coating, and the coated hair was wound around a permanent rod, and 40 ml of Permanent composition 3 was further applied to the wound hair for coating by the spray gun, and then an irradiation of a far infrared ray was conducted with relatively high humidity for 10 to 12 minutes. Intermediate rinse (60 to 100ml) was applied to the hair for coating, and the rinse-treated hair was coated with 40ml of Oxidizing agent 1 to stand for 5 minutes, and the hair was further coated with 40ml of Oxidizing agent 1. After allowing to stand for 5 minutes, the treated hair was washed by water. As a result, although the permanent-treated hair firmly formed a wave configuration, the shapability between the hair tips and the hair roots was different from each other and the hair was dry and loose slightly.

### Comparative Example 2

A virgin hair of 10's female was washed with Shampoo 2, and the washed hair was hair-cut. Subsequently, 40 ml of Permanent composition 4 was applied to the hair for coating by a spray gun, and the coated hair was irradiated with a far infrared ray for 10 to 15 minutes, and then the hair was wound on a permanent rod. The wound hair was further coated with 40 ml of Permanent composition 4 by a spray gun, and irradiated by the far infrared ray for 15 to 20 minutes. The treated hair was coated with 60 to 100ml of Intermediate rinse, and 40ml of Oxidizing agent 1 was applied to the rinse-treated hair for coating. After allowing to stand for 5 minutes, further 40ml of Oxidizing agent 1 was applied to the hair for coating, and the coated hair was left for 5 minutes, and the treated hair was washed by water. The permanent-treated hair gave a weak shapability for waving, and the wave immediately collapsed.

### Example 4

To a damaged hair by dyeing of 50's female, 15 to 20 ml of Cuticle-conditioning agent was sprayed on damaged parts of the hair, and the treated hair was dried. The dried hair was washed with 3 to 5ml of Shampoo 1, and then further washed by 3 to 5ml of Shampoo 1 with fully lathering up. Subsequently, 1ml of Treatment 1 was applied to the washed hair for coating, and the coated hair was towel-blotted to dry without rinsing, and then the hair was hair-cut. The hair was coated with 12ml of Treatment 1 again, and 100ml of water was sufficiently sprayed to the coated hair, and 10 to 20 ml of Cuticle-conditioning agent was then sprayed to the wetted hair. The treated hair was warmed with a mist or a steamer for 10 minutes. Subsequently, the hair was coated with 40 ml of a mixed composition of Permanent composition 2 (prepared by warming at 35 °C) and Treatment 1 in a ration of the former/the latter = 4/1 (volume ratio), and the coated hair was wound on a permanent rod. The wound hair was sprayed with 20ml of water, and covered with a cap, and then was allowed to stand for 10 minutes. The treated hair was further coated with 40 ml of Permanent composition 2 (prepared by warming at 35 °C), and the coated hair was covered with a cap, and then allowed to stand for 5 to 25 minutes. The curl was confirmed with visual confirmation by releasing one of rods from the hair. Subsequently, after 40 ml of water was slightly sprayed to the hair, the wetted hair was slightly blotted with a towel. The hair was coated with 40ml of Oxidizing agent 2. After allowing to stand for 5 minutes, 40ml of Oxidizing agent 2 was further applied to the hair for coating, and the coated hair was left for 5 to 7 minutes . Subsequently, 60 to 100ml of Rinse was applied to the hair for coating, and after allowing to stand for an appropriate time, the permanent rods was removed from the treated hair. Lastly, 15 to 20ml of Rinse was applied to the hair for coating, and the rinse-treated hair was slightly washed with water. As the results, the permanent hair had a slightly wet texture to give a pleasant touch-feeling, and the permanent wave was firmly formed from the hair roots to the hair tips all over.

### Comparative example 3

A hair damaged by dyeing of 50's aged female was washed with Shampoo 2, and the washed hair was hair-cut. Subsequently, 12 ml of Treatment 2 was applied to the hair for coating, and the coated hair was wound on permanent rods with wetting the hair by water. Subsequently, water was sprayed to the wound hair, and the wetted hair was coated with 40 ml of Permanent composition 3. After allowing to stand for 1 to 2 minutes, the treated hair was coated successively with 60 to 100ml of Intermediate rinse and 40ml of Oxidizing agent 1, and the coated hair was allowed to stand for 10 minutes . Further 40ml of Oxidizing agent 1 was applied to the treated hair for coating, and allowed to stand for 5 minutes, and then the hair was washed by water. Lastly, 12 ml of Treatment 2 was applied to the hair for coating again. The resulting permanent-treated hair was further damaged, and the tips of the hair entangled or dissolved and were enable to be combed entirely. Moreover, the wave was irregularly formed, and waves of the tips of the hair were not formed.

### Comparative example 4

A damaged hair by dyeing of 50's female was washed with Shampoo 2, and the washed hair was hair-cut. Subsequently, 12 ml of Treatment 2 was applied to the hair for coating, and the coated hair was wound on permanent rods with wetting the hair by water, and the wound hair was sprayed with water. The wound and wetted hair was coated with 40 ml of Permanent composition 4, and then the coated hair was allowed to stand for 5 to 8 minutes. The hair was coated with 60 to 100ml of Intermediate rinse, and 40ml of Oxidizing agent 1 was applied to the rinse-treated hair for coating. After allowing to stand for 5 minutes, the hair was further coated with 40ml of Oxidizing agent 1, and the coated hair was allowed to stand for 5 minutes. Furthermore, 40ml of Oxidizing agent 1 was applied to the hair for coating, and the treated hair was washed by water. As a result, the permanent-treated hair had a slightly pleasant touch-feeling, but the permanent-treated hair gave limp and sticky. Moreover, a waved hair was not firmly formed.

### Example 5

A damaged short hair of 70's female was washed with 3 to 5ml of Shampoo 1, and the washed hair was further washed by 3 to 5ml of Shampoo 1 with fully lathering up. Subsequently, 1ml of Treatment 1 was applied to the hair for coating, the coated hair was towel-blotted to dry without rinsing of Treatment 1, and then the hair was hair-cut. The hair was coated with 3ml of Treatment 1 again, and water was sufficiently sprayed to the coated hair. Subsequently, 10 to 20 ml of Cuticle-conditioning agent was sprayed to the wetted hair, and then the treated hair was warmed with a mist or a steamer for 10 minutes. Subsequently, the hair was coated with 40 ml of a mixed composition of Permanent composition 2 (prepared by warming at 35 °C) and Treatment 1 in a ratio of the former/the latter = 4/1 (volume ratio), and the coated hair was wound on a permanent rod, and then 20ml of water was sprayed to the wound hair. The wetted hair was further coated with 40 ml of Permanent composition 2 (prepared by warming at 35 °C), and the coated hair was covered with a cap and was allowed to stand for 5 to 25 minutes. The curl was confirmed with visual confirmation by releasing one of rods from the hair. Subsequently, 40ml of Oxidizing agent 2 was applied to the hair for coating, and the coated hair was allowed to stand for 5 minutes. The hair was coated with further 40ml of Oxidizing agent 2, and was allowed to stand for 5 to 7 minutes. Rinse (60 to 100ml) was applied to the hair for coating, and the rinse-treated hair was allowed to stand for an appropriate time, and then the permanent rods were taken off from the hair. The resulting hair was risen from the hair roots to the hair tips, and the whole of the hair gave gloss and elasticity. Moreover, after repeating a permanent treatment 3 times or more, the damaged hair became thicker soundly.

### Comparative example 5

A damaged short hair of 70's female was washed with Shampoo 2, and the washed hair was hair-cut. Subsequently, 3 ml of Treatment 2 was applied to the hair for coating, and the coated hair was wound on a permanent rod with wetting the hair by water, and then the wound hair was sprayed with water. The wetted hair was coated with 40 ml of Permanent composition 3, and then the coated hair was allowed to stand for 5 to 8 minutes. The curl was confirmed with visual confirmation by releasing one of rods from the hair. After the confirmation, the hair was coated successively with 60 to 100ml of Intermediate rinse and 40ml of Oxidizing agent 1. After allowing to stand for 5 minutes , 40ml of Oxidizing agent 1 was further applied to the hair for coating. The coated hair was allowed to stand for 5 minutes, and then the hair was washed by water. The resulting hair lost elasticity, and the tips of the hair was dry and loose to be unable to comb with fingers. Moreover, after repeating a permanent treatment 2 times or more, the hair became thinner.

### Comparative example 6

A damaged short hair of 70's female was washed with shampoo 2, and the washed hair was hair-cut. Subsequently, 3 ml of Treatment 2 was applied to the hair for coating, and the coated hair was wound on permanent rods with wetting the hair by water. The wound hair was sprayed with water, and 40 ml of Permanent composition 4 was applied to the wetted hair for coating, and then the coated hair was allowed to stand for 5 to 12 minutes. The curl was confirmed with visual confirmation by releasing one of rods from the hair. After the confirmation, the hair was coated successively with 60 to 100ml of Intermediate rinse and 40ml of Oxidizing agent 1. After allowing to stand for 5 minutes, the hair was further coated with 40ml of Oxidizing agent 1, and the coated hair was allowed to stand for 5 minutes. The hair was furthermore coated with 40ml of Oxidizing agent 1, and then the coated hair was washed by water. As a result, the wave was not formed on the roots of the permanent-treated hair, and the whole wave became collapsed. Moreover, the whole hair had no rigidity, and the hair became thinner after permanent treatments of not less than 5 to 6 times.

### Example 6

The permanent processing was conducted in the same manner as Example 1 except for using Permanent composition 5 instead of Permanent composition 1. An excellent curl was formed in the same shorter irradiation time of a far infrared ray as in Example 1.

## Claims

1. A permanent composition comprising a ceramide and cysteine.

2. A permanent composition according to claim 1, wherein the ratio of is 1/1000 to 5/1 (weight ratio) as a ratio of the ceramide/the cysteine.

3. A permanent composition according to claim 1, which comprises 0.001 to 5 % by weight of the ceramide and 1 to 10 % by weight of cysteine.

4. A permanent composition according to claim 1, which further comprises a phospholipid.

5. A permanent composition according to claim 4, wherein the ratio of the ceramide to the phospholipid is 1/0.002 to 1/15000 (weight ratio) as a ratio of the ceramide/the phospholipid.

6. A permanent composition according to claim 4, wherein the content of the phospholipid is 0.001 to 15 % by weight.

7. A set of permanent treating agents comprising a permanent composition recited in claim 1 and at least one treating agent selected from the group consisting of a shampoo, a treatment, an oxidizing agent, a rinse and a cuticle-conditioning agent, wherein the treating agent comprises a ceramide.

8. A set of permanent treating agents according to claim 7, wherein the treating agent further comprises a phospholipid.

9. A process for producing a permanent composition, which comprises aging an aqueous composition comprising a ceramide and cysteine.

10. A process for producing a permanent composition according to claim 9, wherein the aqueous composition is warmed at 25 to 50 °C.

11. A permanent process which comprises applying a permanent composition recited in claim 1 to a hair.

12. A permanent process according to claim 11, which comprises washing a hair with a shampoo comprising a ceramide, and applying the permanent composition to the hair to form a shaped or configured hair.

13. A permanent process according to claim 11, wherein the permanent composition and a treatment comprising a ceramide are applied to a hair for the formation of the hair.

14. A permanent process according to claim 12 or 13, wherein the permanent composition is applied to a hair after forming the hair in the form of a desired shape or configuration.

15. A permanent process according to claim 12 or 13, which comprises a step for washing a hair with a shampoo comprising a ceramide, a step for applying a treatment comprising a ceramide to the hair, a step for applying the permanent composition to the hair for forming the hair in the form of a desired shape or configuration, and a step for treating the hair with an oxidizing agent.
